# EUROPEAN PATENT APPLICATION

(11) **EP 1 688 035 A1**
(43) Date of publication of application: **09.08.2006**
(21) Application number: 04787744.4
(22) Date of filing: 08.09.2004
(51) Int. Cl.: A01K 67/027, G01N 33/15, C12N 15/12

(54) **HYPERLIPEMIA/ HYPERALBUMINEMIA MODEL ANIMAL**

(30) Priority: 04.11.2003 JP 2003374098
(71) Applicant: Japan Science and Technology Agency, Kawaguchi-shi, Saitama 332-0012 (JP)
(72) Inventor: YAMAGUCHI, Masayoshi, Sizuoka-shi, Shizuoka 4200913 (JP)
(74) Representative: van Westenbrugge, Andries
(86) International application number: PCT/JP2004/013061
(87) International publication number: WO 2005/041648

(57) **Abstract**

The present invention provides a hyperlipemia and/or hyperalbuminemia animal model that is useful for the development of preventive/therapeutic drugs for hyperlipemia and hyperalbuminemia, and in particular, that develops hyperlipemia and hyperalbuminemia at the stage of senility (advanced age) (in case of human, middle-aged and elderly). The hyperlipemia and/or hyperalbuminemia animal model is obtained by raising a transgenic rat (homozygote) into which a regucalcin gene is introduced and which overexpresses regucalcin to the stage of senility (advanced age), for example, 36 weeks of age, at which it exhibits symptoms of hyperlipemia and/or hyperalbuminemia. This animal model exhibits not only significant and marked increase of serum albumin, HDL-cholesterol and triglyceride concentrations but also bone disorders.

SEQUENCE LISTING

<110> JAPAN SCIENCE AND TECHNOLOGY AGENCY

<120> Model animal with hyperlipidemia and hyperalbuminemia

<130> YG2004-17PCT

<150> JP2003-374098
<151> 2003-11-04

<160> 4

<170> PatentIn Ver. 2.1

<210> 1
<211> 900
<212> DNA
<213> Rattus norvegicus

<220>
<221> CDS
<222> (1)..(900)

<400> 1
atg tct tcc atc aag att gaa tgt gtt tta agg gag aac tac agg tgt 48
Met Ser Ser Ile Lys Ile Glu Cys Val Leu Arg Glu Asn Tyr Arg Cys
1               5                   10                  15

ggg gag tcc cct gtg tgg gag gag gca tca aag tgt ctg ctg ttt gta 96
Gly Glu Ser Pro Val Trp Glu Glu Ala Ser Lys Cys Leu Leu Phe Val
            20                  25                  30

gac atc cct tca aag act gtc tgc cga tgg gat tcg atc agc aat cga 144
Asp Ile Pro Ser Lys Thr Val Cys Arg Trp Asp Ser Ile Ser Asn Arg
        35                  40                  45

gtg cag cga gtt ggt gta gat gcc cca gtc agt tca gtg gca ctt cga 192
Val Gln Arg Val Gly Val Asp Ala Pro Val Ser Ser Val Ala Leu Arg
    50                  55                  60

cag tca gga ggc tat gtt gcc acc att gga acc aag ttc tgt gct ttg 240
Gln Ser Gly Gly Tyr Val Ala Thr Ile Gly Thr Lys Phe Cys Ala Leu
65                  70                  75                  80

aac tgg gaa gat caa tca gta ttt atc cta gcc atg gtg gat gaa gat 288
Asn Trp Glu Asp Gln Ser Val Phe Ile Leu Ala Met Val Asp Glu Asp
                85                  90                  95

aag aaa aac aat cga ttc aat gat ggg aag gtg gat cct gct ggg aga 336
Lys Lys Asn Asn Arg Phe Asn Asp Gly Lys Val Asp Pro Ala Gly Arg
            100                 105                 110

tac ttt gct ggt acc atg gct gag gaa acc gcc cca gct gtt ctg gag 384
Tyr Phe Ala Gly Thr Met Ala Glu Glu Thr Ala Pro Ala Val Leu Glu
        115                 120                 125

cgg cac caa ggg tcc ttg tac tcc ctt ttt cct gat cac agt gtg aag 432
Arg His Gln Gly Ser Leu Tyr Ser Leu Phe Pro Asp His Ser Val Lys
    130                 135                 140

aaa tac ttt aac caa gtg gat atc tcc aat ggt ttg gat tgg tcc ctg 480
Lys Tyr Phe Asn Gln Val Asp Ile Ser Asn Gly Leu Asp Trp Ser Leu
145                 150                 155                 160

gac cat aaa atc ttc tac tac att gac agc ctg tcc tac act gtg gat 528
Asp His Lys Ile Phe Tyr Tyr Ile Asp Ser Leu Ser Tyr Thr Val Asp
                165                 170                 175

gcc ttt gac tat gac ctg cca aca gga cag att tcc aac cgc agg act 576
Ala Phe Asp Tyr Asp Leu Pro Thr Gly Gln Ile Ser Asn Arg Arg Thr
            180                 185                 190

gtt tac aag atg gaa aaa gat gaa caa atc cca gat gga atg tgc att 624

Val Tyr Lys Met Glu Lys Asp Glu Gln Ile Pro Asp Gly Met Cys Ile
        195                 200                 205

gat gtt gag ggg aag ctt tgg gtg gcc tgt tac aat gga gga aga gta     672
Asp Val Glu Gly Lys Leu Trp Val Ala Cys Tyr Asn Gly Gly Arg Val
    210                 215                 220

att cgc cta gat cct gag aca ggg aaa aga ctg caa act gtg aag ttg     720
Ile Arg Leu Asp Pro Glu Thr Gly Lys Arg Leu Gln Thr Val Lys Leu
225                 230                 235                 240

cct gtt gat aaa aca act tca tgc tgc ttt gga ggg aag gat tac tct     768
Pro Val Asp Lys Thr Thr Ser Cys Cys Phe Gly Gly Lys Asp Tyr Ser
                245                 250                 255

gaa atg tac gtg aca tgt gcc agg gat ggg atg agc gcc gaa ggt ctt     816
Glu Met Tyr Val Thr Cys Ala Arg Asp Gly Met Ser Ala Glu Gly Leu
            260                 265                 270

ttg agg cag cct gat gct ggt aac att ttc aag ata aca ggt ctt ggg     864
Leu Arg Gln Pro Asp Ala Gly Asn Ile Phe Lys Ile Thr Gly Leu Gly
        275                 280                 285

gtc aaa gga att gct cca tat tcc tat gca ggg taa                     900
Val Lys Gly Ile Ala Pro Tyr Ser Tyr Ala Gly
    290                 295                 300


<210> 2
<211> 299
<212> PRT
<213> Rattus norvegicus

<400> 2
Met Ser Ser Ile Lys Ile Glu Cys Val Leu Arg Glu Asn Tyr Arg Cys
1               5                   10                  15
Gly Glu Ser Pro Val Trp Glu Glu Ala Ser Lys Cys Leu Leu Phe Val
            20                  25                  30
Asp Ile Pro Ser Lys Thr Val Cys Arg Trp Asp Ser Ile Ser Asn Arg
        35                  40                  45
Val Gln Arg Val Gly Val Asp Ala Pro Val Ser Ser Val Ala Leu Arg
    50                  55                  60
Gln Ser Gly Gly Tyr Val Ala Thr Ile Gly Thr Lys Phe Cys Ala Leu
65                  70                  75                  80
Asn Trp Glu Asp Gln Ser Val Phe Ile Leu Ala Met Val Asp Glu Asp
                85                  90                  95
Lys Lys Asn Asn Arg Phe Asn Asp Gly Lys Val Asp Pro Ala Gly Arg
            100                 105                 110
Tyr Phe Ala Gly Thr Met Ala Glu Glu Thr Ala Pro Ala Val Leu Glu
        115                 120                 125
Arg His Gln Gly Ser Leu Tyr Ser Leu Phe Pro Asp His Ser Val Lys
    130                 135                 140
Lys Tyr Phe Asn Gln Val Asp Ile Ser Asn Gly Leu Asp Trp Ser Leu
145                 150                 155                 160
Asp His Lys Ile Phe Tyr Tyr Ile Asp Ser Leu Ser Tyr Thr Val Asp
                165                 170                 175
Ala Phe Asp Tyr Asp Leu Pro Thr Gly Gln Ile Ser Asn Arg Arg Thr
            180                 185                 190
Val Tyr Lys Met Glu Lys Asp Glu Gln Ile Pro Asp Gly Met Cys Ile
        195                 200                 205
Asp Val Glu Gly Lys Leu Trp Val Ala Cys Tyr Asn Gly Gly Arg Val
    210                 215                 220
Ile Arg Leu Asp Pro Glu Thr Gly Lys Arg Leu Gln Thr Val Lys Leu
225                 230                 235                 240
Pro Val Asp Lys Thr Thr Ser Cys Cys Phe Gly Gly Lys Asp Tyr Ser
                245                 250                 255
Glu Met Tyr Val Thr Cys Ala Arg Asp Gly Met Ser Ala Glu Gly Leu
            260                 265                 270
Leu Arg Gln Pro Asp Ala Gly Asn Ile Phe Lys Ile Thr Gly Leu Gly
        275                 280                 285
Val Lys Gly Ile Ala Pro Tyr Ser Tyr Ala Gly
    290                 295

<210> 3
<211> 24
<212> DNA
<213> Artificial Sequence

<220>
<223> Description of Artificial Sequence:Primer huRC-1

<400> 3
ggaggctatg ttgccaccat tgga                                              24


<210> 4
<211> 23
<212> DNA
<213> Artificial Sequence

<220>
<223> Description of Artificial Sequence:Primer huRC-2

<400> 4
ccctccaaag cagcatgaag ttg                                               23

## Description

### Technical Field

The present invention relates to a hyperlipemia and/or hyperalbuminemia animal model, preferably to a hyperlipemia and/or hyperalbuminemia animal model which is a regucalcin-overexpressing transgenic non-human animal raised to the stage of senility (advancedage), andamethod for screening therapeutic/preventive drugs for hyperlipemia and/or hyperalbuminemia with the use of the hyperlipemia and/or hyperalbuminemia animal model, etc.

### Background Art

Ca²⁺ plays a major role in a mechanism wherein peptide hormones bind to receptors on cell membranes and transmit their information into cells. In cells, there are many proteins that bind to Ca²⁺, and calmodulin plays a pivotal role as a protein that enhances the effect. It has been revealed that Ca²⁺ binds to such calmodulin and activates various enzymes involved in the regulationof cellular functions (Science, 202, 19-27, 1984) . In addition, it is known that Ca²⁺ has an effect on protein kinase C and other Ca²⁺-binding proteins (including enzymes) (Science, 233, 305-312, 1986) . Regucalcin is also a Ca²⁺-binding protein isolated from rat hepatic cytoplasm by the present inventors.

Regucalcin is a Ca²⁺-binding protein whose molecular weight is 33388 and whose Ca²⁺-binding constant is 4.19 × 10⁵ M⁻¹, which has 6 to 7 high-affinity Ca²⁺-binding sites and contains 34% of α-helical structure, and is an acidic protein with isoelectric point pI 5.20, prominently present in the liver. Regucalcin is a unique protein that does not contain EF hand motif (domain), a site observed in calmodulin and many other Ca²⁺-binding proteins. For example, in calmodulin, binding to Ca²⁺ increases the α-helical content and makes the structure firm, whereas in regucalcin, it decreases the α-helical content. On the other hand, in the regulation of cellular functions, it has been revealed that regucalcin inhibits the calmodulin-induced activation of enzymes and also inhibits the activation of protein kinase C. As aforementioned, much knowledge, such as that regucalcin acts as a regulatory protein of signaling, has been accumulated (FEBS Lett, 327, 251-255, 1993).

Regucalcin genes are localized on chromosome X (Xq 11.1-12) in rat, and on chromosome X in human as well. In addition to in rat and human, regucalcin genes have been observed in higher animals such as monkey, mouse, dog, bovine, rabbit, and chicken, but not in yeast, and are considered to encode highly differentiated proteins. Regucalcin cDNA has been cloned and its whole structure has been determined (Japanese Laid-Open Patent Application No. 7-123985). In case of regucalcin cDNA from rat liver, base pairs encoding total amino acids are 0.897 kb, and 299 amino acids are translated. Further, base sequences of regucalcin cDNA from mouse liver and human liver have been determined as well, and have 94% homology and about 89% homology in comparison to regucalcin cDNA from rat liver, respectively. Expression of regucalcin mRNA is observed in livers of human, rat, mouse, bovine, chicken, etc., and the presence of regucalcin protein is also confirmed in these livers.

It is known that regucalcin is a protein having characteristics as a multifunctional regulatory protein of intracellular Ca²⁺ signaling and is an important protein involved in the regulation of cellular functions (Life Sciences 66, 1769-1780, 2000; Biochemical and Biophysical Research Communications 276, 1-6, 2000). In addition, it has been revealed from animal experiments that in vivo expression of regucalcin in the liver or the kidney decreases in case of hepatic disorders (Molecular and Cellular Biochemistry 131, 173-179, 1994) or renal disorders (Molecular and Cellular Biochemistry 151, 55-60, 1995), suggesting the relation between regucalcin and causes of the diseases. Further, it is known that the method for differentiating sera taken frompatients with hepatic disease by measuring the serum concentration of regucalcin, which is specifically present in the liver unlike the existing hepatic function markers such as GOT and GPT, is useful, in other words, it is known that the measurement is useful as means for differentiating sera taken from patients with hepatic disease because regucalcin increases significantly in the sera taken from patients with hepatic disease, whereas regucalcin is hardly detected in the sera taken from healthy people (Japanese Laid-Open Patent Application No. 10-26623).

As mentioned above, regucaltin protein is a unique multifunctional protein: it is expressed specifically in the liver, and also expressed in the kidney, the heart, the cerebrum (neurons) at a low level, and is involved in the regulation of cellular functions related to intracellular Ca²⁺ signaling, and its decreased expression causes physiological abnormalities. The functional analyses of regucalcin have been conducted with the use of proteins isolated from rat liver, and anti-regucalcin monoclonal antibodies. In addition to the role as a regulatory factor of calcium signal mentioned above, many functional roles of regucalcin in biological regulation have been revealed by the present inventors. Example of such roles include: the regulation of intracellular calcium transport enzymes; a role as a protease-activating factor; the regulation of cell nuclear function including the regulation of calcium transport of cell nuclei, a role in cell nuclear DNA degradation, and a role in cell nuclear function at hepatic regeneration; and a role in renal tubular calcium reabsorption.

In the research process for elucidating various functional roles of regucalcin, the present inventors have focused on the point that regucalcin has specific effects different from those of many other Ca²⁺-binding proteins. The present inventors have assumedthat functional regulation of various cells that involves calcium rests on the balance between the expression amount of regucalcin and of many other Ca²⁺-binding proteins including calmodulin in living bodies, and examined the changes/effects that occur in the living bodies when the balance between the expression amount of regucalcin and of many other Ca²⁺-binding proteins is disrupted, by preparing transgenic rats. The transgenic rats were constructed according to the following steps: regucalcin cDNA was cloned from rat hepatic cDNA library and cDNA encoding the full length of regucalcin protein was isolated; ORF was cut out from the rat regucalcin full length cDNA and introduced into an expression vector (pCXN2) ; this gene expression vector was microinjected into the male nucleus of a rat fertilized egg; the fertilized egg was transplanted into an oviduct of a foster parent rat to generate a baby rat; DNAs were extracted from baby rats thus generated and it was confirmed whether regucalcin cDNAs were incorporated into the rats by PCR; and 5 homozygous rats (4 males, and 1 female) expressing regucalcin cDNA were found from 29 baby rats. It has been reported that: the weight gain of the transgenic rats is significantly suppressed; when evaluation of bone morphological (bone density, bone strength, diaphyseal cortical thickness, cortical circumference) measurement with a pQCT (peripheral Quantitative Computed Tomography) bone-density measuring equipment for animal research, and of biochemical (calcium content, activity of alkaline phosphatase, which is a marker enzyme of osteoblast/bone-forming cells, DNA content, which is an index of cell count in bone tissues) measurement of bone components were conducted for the transformed rats which had acquired an ability to overexpress regucalcin by the introduction of regucalcin genes mentioned above and which exhibited no bone disorder apparently, morphologically and biochemically prominent bone disorders, such as weakening of bone tissues caused by bone resorption (bone mineral dissolution) due to bone loss and decreased bone density, morphological changes in bones, growth retardation of coccyx, were exhibited particularly in the femur; the characteristics of the regucalcin-overexpressing rat model of disease are transgenerationally stable so that the rat model can stand commercial production (Japanese Laid-Open Patent Application No. 2003-164238).

On the other hand, hyperlipemia is a clinical condition which shows increased serum lipid concentrations such as cholesterol and triglyceride, and is closely related to the development of circulatory diseases including arteriosclerosis, hypertension, and stroke. In addition, hyperalbuminemia is a clinical condition which shows increased serum albumin concentration synthesized in the liver, and is related to various hepatic diseases. As for techniques regarding to hyperlipemia animal models mentioned above, the following models are known: hyperlipemic rat useful as an animal model for human focal glomerulosclerosis (K. Yamasaki and Y. Yoshikawa, Laboratory Animal Science A4 (2) 1994, 125-130); non-human mammal having DNA into which an exogenous 25-hydroxyvitamin D₃ 24-hydroxylase gene is incorporated, and exhibiting diseases caused by disorders of vitamin D₃ metabolism including renal diseases, bone diseases, joint diseases, pulmonary diseases, hyperlipemia, arteriosclerosis, cardiac diseases, diabetes, obesity, gastrointestinal diseases, infections, allergies, endocrine diseases, dementia, cancers (Japanese Laid-Open Patent Application No. 11-9140); animal model of disease useful as a treatment model for hyperlipemia, obtained by using embryonic stem cells of non-human mammals whose LDL (low density lipoproteins) receptor genes are inactivated (Japanese Laid-Open Patent Application No. 10-56915); diabetic (NIDDM) rat (ZDF/Gmi-fa/fa; Charles River Laboratories Japan, Inc.); SDF (Spontaneously Diabetic Torii) rat (Torii Pharmaceutical Co., Ltd.); WHHL rabbit (Kitayama Labes Co., Ltd. /Oriental Yeast Co., Ltd.)

The object of the present invention is to provide a hyperlipemia and/or hyperalbuminemia animal model useful for the development of preventive/therapeutic drugs for hyperlipemia and hyperalbuminemia, which are rapidly increasing with the recent lifestyle changes, particularly a hyperlipemia and/or hyperalbuminemia animal model that develops hyperlipemia and hyperalbuminemia at the stage of senility (advanced age) (in case of human, middle-aged and elderly), and a method for screening preventive/therapeutic drugs for hyperlipemia and/or hyperalbuminemia using the hyperlipemia and/or hyperalbuminemia animal model.

The regucalcin-overexpressing transgenic rats already developed by the present inventors show the onset of osteoporosis during the growth period, at 5 weeks of age, and are valued as a useful animal model, and have already come onto the market. The regucalcin transgenic rats were raised to the stage of senility (advanced age) (in case of human, middle-aged and elderly), and the changes in biological functions were examined. By the dissection of aged rats (36 weeks of age) (9 months of age), significantly and markedly increased concentrations of serum albumin, HDL-cholesterol, and triglyceride were newly observed in addition to bone loss and increased concentration of serum inorganic phosphorus, which were known hithereto in growing rats, and thus it has been revealed that the regucalcin transgenic rats develop hyperalbuminemia and hyperlipemia at the stage of advanced age. As serum albumin, HDL-cholesterol and triglyceride are produced in and released from the liver, the aged regucalcin transgenic rats were considered to have developed hepatic diseases. In fact, when the rats were dissected, it was observed that the livers of the regucalcin transgenic rats exhibited fatty liver-like conditions, in comparison to the livers of normal rats.

Subsequently, age-related changes in the expression of hyperlipemia/hyperalbuminemia were examined with the use of 14-, 25-, 36-, 50-week-old transgenic rats. As a result, it was found that female rats that were 14 weeks of age or older indicated elevated levels of serum lipid concentrations (free fatty acid, triglyceride, HDL-cholesterol, free cholesterol), and revealed that the elevation was prominent in 50-week-old (1-year-old) rats. On the other hand, it was revealed that female rats that were 25 weeks of age or older indicated elevated levels of serum albumin concentration.
The present invention has been completed based on these findings.

### Disclosure of the Invention

The present invention relates to: (1) a hyperlipemia and/or hyperalbuminemia animal model comprising a transgenic non-human animal into which a regucalcin gene is introduced and which overexpresses regucalcin; (2) the hyperlipemia and/or hyperalbuminemia animal model according to (1), which is obtained by raising the transgenic non-human animal to the stage of senility (advanced age) at which it exhibits a symptom of hyperlipemia and/or hyperalbuminemia; (3) the hyperlipemia and/or hyperalbuminemia animal model according to (1), which is obtained by raising the transgenic non-human animal (female) until it exhibits a symptom of hyperalbuminemia; (4) The hyperlipemia and/or hyperalbuminemia animal model according to any one of (1) to (3), wherein the non-human animal exhibits a bone disorder at the stage of senility (advanced age); (5) the hyperlipemia and/or hyperalbuminemia animal model according to any one of (1) to (4), which is a homozygote; and (6) the hyperlipemia and/or hyperalbuminemia animal model according to any one of (1) to (5), wherein the non-human animal is a rat.

The present invention also relates to: (7) the hyperlipemia and/or hyperalbuminemia animal model according to (6), wherein the stage of senility (advanced age) means 36 to 50 weeks of age; (8) a method for using a transgenic non-human animal into which a regucalcin gene is introduced and which overexpresses regucalcin as an animal model for hyperlipemia and/or hyperalbuminemia; (9) the method according to (8), wherein the transgenic non-human animal is raised to the stage of senility (advanced age) and used as an animal model for hyperlipemia and/or hyperalbuminemia; (10) the method according to (8), wherein the transgenic non-human animal (female) is raised until it exhibits a symptom of hyperalbuminemia and used as an animal model for hyperlipemia and/or hyperalbuminemia; (11) the method according to any one of (8) to (10), wherein the non-human animal exhibits a bone disorder at the stage of senility (advanced age); and (12) the method according to any one of (8) to (11), which is a homozygote.

The present invention further relates to: (13) the method according to any one of (8) to (12), wherein the non-human animal is a rat; (14) the method according to (13), wherein the stage of senility (advanced age) means 36 to 50 weeks of age; (15) a method for screening a therapeutic drug for hyperlipemia and/or hyperalbuminemia comprising the steps of; administering a test substance to the hyperlipemia and/or hyperalbuminemia animal model according to any one of (1) to (7), andmeasuring/evaluating the amount of lipid and/or albumin in blood; and (16) a method for screening a preventive drug for hyperlipemia and/or hyperalbuminemia comprising the steps of; administering a test substance to the hyperlipemia and/or hyperalbuminemia animal model according to any one of (1) to (7) before it reaches the stage of senility (advanced age) at which it exhibits a symptom of hyperlipemia and/or hyperalbuminemia, and measuring/evaluating the amount of lipid and/or albumin in blood after it reaches the stage of senility (advanced age).

### Brief Description of Drawings

[Fig.1] Fig. 1 is a view showing the measurement results of serum calcium of 36-week-old regucalcin transgenic rats of the present invention.
[Fig.2] Fig. 2 is a view showing the measurement results of serum inorganic phosphorus of 36-week-old regucalcin transgenic rats of the present invention.
[Fig.3] Fig. 3 is a view showing the measurement results of serum zinc of 36-week-old regucalcin transgenic rats of the present invention.
[Fig.4] Fig. 4 is a view showing the measurement results of serum glucose of 36-week-old regucalcin transgenic rats of the present invention.
[Fig.5] Fig. 5 is a view showing the measurement results of serum triglyceride of 36-week-old regucalcin transgenic rats of the present invention.
[Fig.6] Fig. 6 is a view showing the measurement results of serum HDL-cholesterol of 36-week-old regucalcin transgenic rats of the present invention.
[Fig.7] Fig. 7 is a view showing the measurement results of serum albumin of 36-week-old regucalcin transgenic rats of the present invention.
[Fig.8] Fig. 8 is a view showing the measurement results of body weight of 36-week-old regucalcin transgenic rats of the present invention.
[Fig.9] Fig. 9 is a view showing the measurement results of calcium content of femoral-diaphysis and -metaphysis of 36-week-old regucalcin transgenic rats of the present invention.
[Fig.10] Fig. 10 is a view showing the measurement results of DNA content (an index of cell count) of femoral-diaphysis and -metaphysis of 36-week-old regucalcin transgenic rats of the present invention.
[Fig.11] Fig. 11 is a view showing the measurement results of serum free fatty acid of 14-, 25-, 36-, and 50-week-old regucalcin transgenic rats of the present invention.
[Fig.12] Fig. 12 is a view showing the measurement results of serum triglyceride of 14-, 25-, 36-, and 50-week-old regucalcin transgenic rats of the present invention.
[Fig.13] Fig. 13 is a view showing the measurement results of serum HDL-cholesterol of 14-, 25-, 36-, and 50-week-old regucalcin transgenic rats of the present invention.
[Fig.14] Fig. 14 is a view showing the measurement results of serum free cholesterol of 14-, 25-, 36-, and 50-week-old regucalcin transgenic rats of the present invention.
[Fig.15] Fig. 15 is a view showing the measurement results of serum albumin of 14-, 25-, 36-, and 50-week-old regucalcin transgenic rats of the present invention.

### Best Mode of Carrying Out the Invention

The animal model of the present invention is not particularly limited as long as it is a hyperlipemia and/or hyperalbuminemia animal model comprising a transgenic non-human animal into which a regucalcin gene is introduced and which overexpresses regucalcin, and the method of the present invention is not particularly limited as long as it is a method using a transgenic non-human animal into which a regucalcin gene is introduced and which overexpresses regucalcin as an animal model for hyperlipemia and/or hyperalbuminemia. Here, "to overexpress regucalcin" means to express significantly larger amount of regucalcin in comparison to an amount of regucalcin expressed by wild-type non-human animals. Further, examples of the nom-human animal mentioned above include rat, mouse, bovine, porcine, chicken, frog, human, dog, and rabbit, and among them, rat is preferable. In case of mouse, which is commonly used as an animal model, analyses of clinical conditions sometimes have limitations due to the smallness of mouse organs, however, in case of rat, blood pressure measurement can be conducted, for example, and it is very useful as means for animal experiments for elucidating clinical conditions and gene therapy.

As the transgenic non-human animal mentioned above, a transgenic non-human animal introduced with, for example, a linear DNA in which a cytomegalovirus-IE enhancer, a chicken β-actin promoter, a regucalcin gene, a rabbit β-globin poly A signal are arranged in this order, can be exemplified. For instance, with the use of an expression vector (pCXN2) which has a marker gene, a cytomegalovirus-IE enhancer, a chicken β-actin promoter, a cDNA-inserted site, a rabbit β-globin poly A signal, etc., and which is introduced with regucalcin full length cDNA, transgenic non-human animals can be efficiently obtained.

Further, examples of a preferable mode of the transgenic non-human animal mentioned above include: a transgenic non-human animal obtained by raising to the stage of senility (advanced age) at which it exhibits symptoms of hyperlipemia and/or hyperalbuminemia; and a female transgenic non-human animal obtained by raising until it exhibits symptoms of hyperalbuminemia. The examples also include: a transgenic non-human animal whose regucalcin gene is a gene that encodes a protein comprising an amino acid sequence shown by SEQ ID NO: 2 in the sequence listing; and in particular, a transgenic non-human animal whose gene that encodes a protein comprising an amino acid sequence shown by SEQ ID NO: 2 in the sequence listing is a rat regucalcin gene comprising a DNA sequence shown by SEQ ID NO: 1 in the sequence listing. The origin of the regucalcin gene is not particularly limited and examples of the origin include mouse, bovine, porcine, chicken, frog, human, dog, and rabbit as well as rat.

In addition, as a preferable mode of the transgenic non-human animal mentioned above, homozygous transgenic non-human animals can be exemplified. Homozygotes which are homozygous for the mutant chromosome can be obtained by crossing non-human animals such as rats which are heterozygous for the chromosome each other, and are particularly preferable as an experimental animal model because they express a larger amount of regucalcin than heterozygotes do.

Further, as for the transgenic non-human animal mentioned above, transgenic animals which exhibit bone disorders at the stage of senility (advanced age) as well as symptoms of hyperlipemia and/or hyperalbuminemia are preferable. Here, "bone disorder" means abnormal conditions of bone or bone growth such as bone loss, weakening of bone tissues, changes of bone morphology, bone growth retardation, etc., caused by disorders of calcium and bone metabolism as represented by osteoporosis. The non-human animal models which exhibit bone disorders at the stage of senility (advanced age) as well as symptoms of hyperlipemia and hyperalbuminemia can be used for the screening of preventive/therapeutic drugs for hyperlipemia and hyperalbuminemia, and also for the screening of preventive/therapeutic drugs for osteoporosis at the stage of senility (advanced age) .

In a case where the non-human animal is a rat, that is, a transgenic rat which overexpresses regucalcin, the stage of senility (advanced age) can be exemplified by 30 weeks of age or older, preferably, 36 to 50 weeks of age. In case of female transgenic rats, elevated level of serum albumin concentration is observed in rats that are 25 weeks of age or older.

The method for screening a therapeutic drug for hyperlipemia and/or hyperalbuminemia of the present invention is not particularly limited as long as it is a method comprising the steps of; administering a test substance to the hyperlipemia and/or hyperalbuminemia animal model of the present invention mentioned above, and measuring/evaluating the amount of lipid and/or albumin in blood. The method for screening a preventive drug for hyperlipemia and/or hyperalbuminemia of the present invention is not particularly limited as long as it is a method comprising the steps of; administering a test substance to the hyperlipemia and/or hyperalbuminemia animal model of the present invention mentioned above before it reaches the stage of senility (advanced age) at which it exhibits symptoms of hyperlipemia and/or hyperalbuminemia, and measuring/evaluating the amount of lipid and/or albumin in blood after it reaches the stage of senility (advanced age). In addition to known synthetic compounds, peptides, proteins, etc, for example, tissue extracts from mammals, cell culture supernatants, components extracted from various plants are used as test substance. For instance, preventive/therapeutic drugs for hyperlipemia and/or hyperalbuminemia can be screened by orally or parenterally administering a test compound to the hyperlipemia and/or hyperalbuminemia animal model of the present invention, and by conducting the measurement/evaluation of the amount of lipid and/or albumin in blood, or the observation/evaluation of the clinical condition of the liver, in the animal model. Further, when conducting these screenings, it is preferable to evaluate in comparison to the case of wild-type non-human animals, particularly, wild-type non-human animals which are littermates, because precise comparative experiments can be conducted at an individual level.

The present invention will be described more specifically with reference to the Examples, but the technical scope of the present invention is not limited to these exemplifications.

### Example 1

### [Generation of transgenic rat]

### (Preparation of RNA)

Liver was extracted from a male Wistar rat (3 weeks of age) and homogenized in a guanidine-isothiocyanate solution (4M guanidinium thiocyanate, 25 mM sodium citrate (pH 7.0), 0.5% sarcosyl, 0.1 M 2-mercaptoethanol, 2M sodium acetate). The homogenate was subjected to extraction with a mixed solution of phenol-chloroform-isoamyl alcohol, and centrifugation was conducted at 4°C, 10,000 x g, for 20 minutes. Isopropanol was added to the aqueous phase and the resulting mixture was left still at -20°C to precipitate RNA. The precipitate was collected, and dissolved into 0.5% sodium dodecyl sulfate treated with diethylpyrocarbonate. The resultant was made to pass through an oligo (dT) cellulose column to purify poly (A) + RNA.

### (Preparation of cDNA library)

To the purified poly (A) + RNA (5 µg), 50 units of Moloney-Murine Leukemia virus reverse transcriptase and an oligo (dT) 18 primer linker were added to synthesize a single-stranded cDNA. Further, to the synthesized single-stranded cDNA, E. coli ribonuclease H and DNA polymerase I were added to synthesize a double-stranded cDNA. An EcoRI adaptor was added to this, and the resultant was ligated to a phage expression vector (λZAPII) which had been digested with XhoI, EcoRI . In addition, the ligated cDNA was packaged into phages with the use of a packaging extract, and the phages of cDNA library were constructed.

### (Selection of RC cDNA clone)

About 1 × 10⁶ phages from rat liver cDNA library were mixed with E. coli, and the mixture was seeded onto 20 agar plates. After incubation at 42°C for 3.5 hours, nitrocellulose membranes treated with 10 mM isopropylthio β-D-galactoside were placed onto the plates, and incubation was conducted at 37°C for 3.5 hours. The nitrocellulose membranes were blocked, and subsequently incubated with anti-RC rabbit serum (× 200) at room temperature for 2 hours. The membranes were washed and then alkaline phosphatase-conjugated anti-rabbit IgG antibody was added for incubation. The incubated membranes were soaked in a color development solution (0.35 mM nitroblue tetrazolium, 0.4 mM 5-bromo-4-chloro-3-indolyl phosphate) for color development, and RC cDNA-positive plaques were identified.

### (Subcloning into plasmid vector)

A phage vector λZAPII contains the base sequence of pBluescript, a plasmid vector, in its sequence, and an RC cDNA fragment cloned into λZAPII is inserted into this pBluescript. In addition, there exist the replication origin and the replication termination of a helper phage at both ends of pBluescript. Therefore, phages were isolated from the identified plaques, and the phages and R408 helper phages were infected with E. coli SURE. The pBluescript containing the RC cDNA fragment was synthesized in E. coli, and released out of E. coli in the form of helper phages. The phage solution was further infected with E. coli SURE, and replicated in the bacteria as a plasmid having the RC cDNA fragment. This E. coli was seeded onto an LB plate containing 50 µg/ml ampicillin, and ampicillin-resistant colonies were selected.

### (Base sequence determination of cDNA insert)

The entire base sequence of cDNA insert was determined with the use of Sequenase system (US Biochemical). In other words, plasmid DNA was cut with EcoRI, and the fragment was subjected to alkaline denaturation treatment, then a primer was added to the fragment and annealing was conducted. To the resultant, 35S dCTP, 0.1M DTT, an enzyme solution for Sequenase were added, and the resulting mixture was divided into 4 equal parts. Then, ddATP, ddGTP, ddTTP, ddCTP were added to each part, and the resultant was incubated at 37°C for 5 minutes. These were subjectedto acrylamide gel electrophoresis for separation, and to autoradiography to confirm the base sequence. The entire base sequence of regucalcin cDNA is shown in SEQ ID NO: 1. Further, the amino acid sequence obtained is shown in SEQ ID NO: 2. Based on the sequence, the molecular weight of regucalcin was calculated to be 33,388. This value is consistent with the molecular weight calculated on the basis of SDS polyacrylamide electrophoresis of purified regucalcin.

### (Construction of transgene)

A DNA fragment containing all ORFs was cut out with the use of PstI from a plasmid containing rat regucalcin full-length cDNA obtained, RC-900 (glycerol stock; RC-F), a vector pBluescript SK (-). This PstI fragment thus cut out was incorporated into a PstI site of pBluescript II KS (+). Then an EcoRI fragment obtained by cutting out with EcoRI was introduced into an EcoRI site of the expression vector pCXN2 (Clontech) (Gene 108, 193-199, 1991), and a rat regucalcin expression vector RC/pCXN2 was prepared. This RC/pCXN2 was cut with SalI, SfiI, and MluI, and a linearized 3.6 kbp fragment was obtained.

### (Generation of transgenic rat)

Microinjection of the above-mentioned linearized 3.6 kbp DNA fragment solution into a rat fertilized egg at pronuclear stage was conducted as follows: a 4-week-old Sprague-Dawley (SD) female rat was raised in a light-dark cycle for 12 hours (light hours 4:00 - 16:00), at a temperature of about 23°C, and a humidity of about 55%, and the estrous cycle of the female was observed by vaginal smearmethod to choose the day for the hormone treatment. 150 IU/kg of pregnant mare serum gonadotropin (Nippon Zenyaku Kogyo Co., Ltd.; "PMS Zenyaku") was administered intraperitoneally to the female rat for superovulation treatment, and 48 hours later, 150 IU/kg of human placental gonadotropin (Sankyo Yell Yakuhin Co., Ltd. "PUBEROGEN") was administered intraperitoneally. Subsequently, the female rat was intercrossed with a male by cohabiting. 32 hours after the administration of human placental gonadotropin, a fertilized egg at pronuclear stage was collected by tubal perfusion.

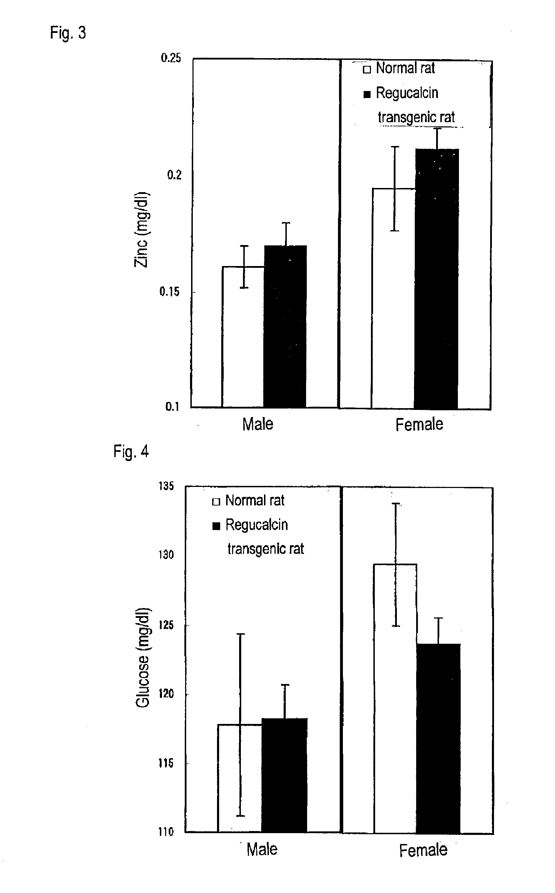

To the male pronucleus of the Wistar rat fertilized egg thus prepared, the above-mentioned 3.6 kbp DNA fragment solution (concentration of 5 ng/µl) was microinjected. The egg to which DNA fragment was injected was cultured overnight by using m-KRB (m-Krebs Ringer buffer solution) medium in a CO₂ incubator. The development proceeded to 2-cell phase on the next day, 2-celled embryos which exhibit no abnormality were transplanted into oviducts of 9 foster parents (pseudopregnant female rats intercrossed with vasoligated male rats), at a rate of 20 to 30 embryos per rat, and 29 newborn rats were obtained. DNAs were collected from tails of 27 rats that survived to 4 weeks of age, and the collected DNAs were examined by PCR with the use of the primer huRC-1; GGAGGCTATGTTGCCACCATTGGA (SEQ ID NO: 3); the primer huRC-2; CCCTCCAAAGCAGCATGAAGTTG (SEQ ID NO: 4) (Fig. 4). As a result, the presence of the transgene was identified in a total of 5 rats (4 males and 1 female) . Among them, 5 rats transmitted the transgene to the next generation.

### Example 2

### [Raising to advanced age and measurement of components]

### (Raising to advanced age)

Among strains of the transgenic rats (heterozygotes) obtained in Example 1, the strains which exhibited the largest amount of regucalcin expression in tail tissues were intercrossed each other to generate transgenic rats (homozygotes) . Further, the rats were identified to be homozygotes by confirming the incorporation of the transgene into genomic DNA extracted from rat tail tissues by PCR, and by detecting more than twice of the incorporated amount compared to the cDNA amount of heterozygotes. The homozygous transgenic rats, 6 males and 6 females, and Spraugue-Dawley (SD) wild-type rats (6 animals), 6 males and 6 females, were raised in an air-conditioned raising room at a temperature of 25°C, fed with solid feed (Oriental Yeast Co., Ltd., MF) at liberty, from birth up to 36 weeks of age.

### (Dissection and measurement items)

Under ether anesthesia, the above-mentioned rats raised to 36 weeks of age were dissected, their blood was collected by cardiopuncture, and their femoral tissues were extracted. The blood was left still for 2.0 minutes at room temperature, and centrifuged at 3000 rpm for 5 minutes to collect serum. The serumwas stored at -32 °C until the measurement of its components. As to the femoral tissues, muscle tissues were exfoliated in a cold 0.25 M sucrose solution, and the femoral tissues were separated into diaphyseal (cortical bone) and metaphyseal (cancellous bone) tissues, then bone marrow was removed by washing, and ossein was obtained. With regard to the serum components, calcium (Calcium C-Test Wako), inorganic phosphorus (P-Test Wako), zinc (Zinc Test Wako), glucose (Glucose Test Wako), triglyceride (Triglyceride Test Wako), HDL-cholesterol (HDL-cholesterol Test Wako), and albumin (Albumin Test Wako) were quantitated respectively by using the measurement kit made by Wako Pure Chemical Industries, Ltd. The calcium content (mg/g bone dry weight) in femoral-diaphyseal and -metaphyseal tissues was measured as follows: diaphysis (corticalbone) and metaphysis (cancellous bone) were dried at 100°C for 6 hours; the weight of the bones was measured; the bones were degraded at 120°C for 24 hours; the fluid volume of the bones was measured; then the bones were dissolved in 6 N hydrochloride to measure bone calcium content by atomic absorbance. The DNA content (an index of cell count) in the bone tissues was measured as follows: diaphysis (cortical bone) and metaphysis (cancellous bone) were soaked in 3 ml of ice-cooled 6.5 mM parbital buffer solution (pH 7.4) respectively; the bones were cut into small pieces, and shaken in 4.0 ml of ice-cooled 0.1 N sodium hydroxide solution for 24 hours for alkaline extraction; subsequently centrifugation was conducted at 10,000 rpm for 5 minutes; supernatant obtained by centrifugation was used to measure DNA content; DNA content was measured according to the method of Ceriotti (J. Biol. Chem., 214, 39-77, 1955). The statistical procedure for testing the significant difference of the experimental results thus obtained was conducted by using Student's t-test.

### (Measurement results of serum components)

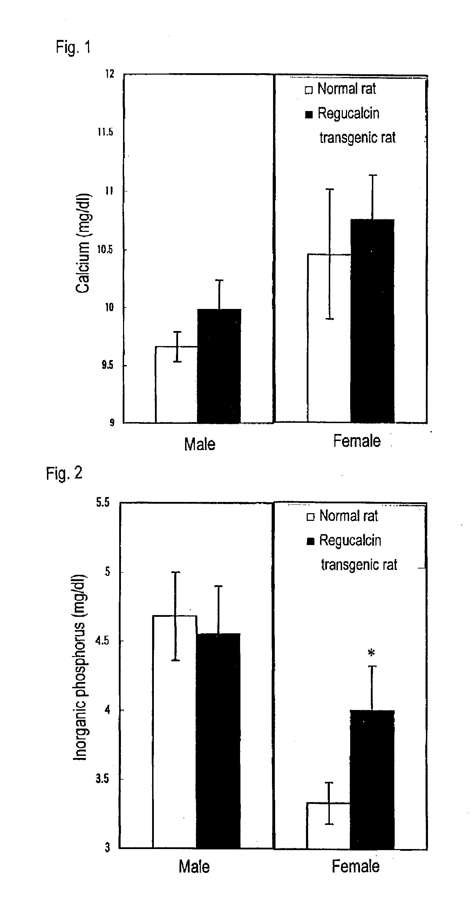

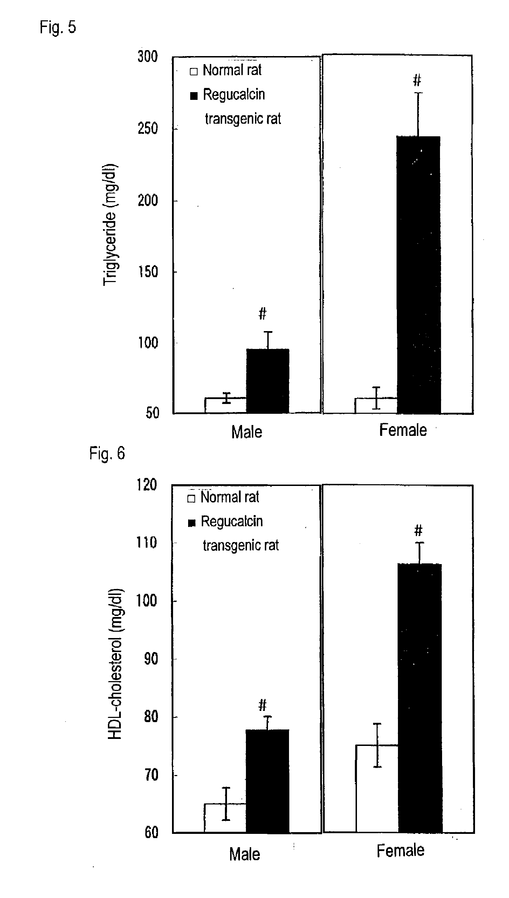

The serum concentrations of calcium, inorganic phosphorus, zinc, glucose, triglyceride, HDL-cholesterol, and albumin were measured respectively, according to the types of rats, transgenic rats (homozygotes) or wild-type rats, and to the sex. The results are shown in Figs. 1 to 7 and Table 1. Each value in Figs. 1 to 7 and Table 1 shows mean value of 6 rats and standard error thereof. Further, in Figs. 1 to 7 and Table 1, each symbol represents: *: P < 0.05 (in comparison to values of the control group), **: P < 0.025 (in comparison to values of the control group), #: P < 0.001 (in comparison to values of the control group). It is seen from Table 1 that the concentrations of inorganic phosphorus (female), triglyceride (male and female), HDL-cholesterol (male and female) and albumin (female) increase significantly, while as those of calcium, zinc, and glucose do not change significantly. In the regucalcin transgenic rats, increase in serum inorganic phosphorus was observed in 5-week-old rats as well, however, increase in serum triglyceride, HDL-cholesterol, and albumin were not known.

**[Table 1]**

| | | Male | | Female | |
|---|---|---|---|---|---|
| | | Normal | Transgenic | Normal | Transgenic |
| Body weight (g) | | 556±12.8 | 534±10.4 | 307±3.6 | 318±11.1 |
| Serum components (mg/dl) | Calcium | 9.66±0.13 | 9.98±0.26 | 10.46±0.56 | 10.76±0.38 |
| | Inorganic phosphorus | 4.68±0.32 | 4.55±0.35 | 3.33±0.15 | 4.00±0.32* |
| | Zinc | 0.161±0.009 | 0.170±0.010 | 0.195±0.018 | 0.212±0.009 |
| | Glucose | 117.8±6.6 | 118.2±2.5 | 129.4±4.4 | 123.7±1.9 |
| | Triglyceride | 60.9±3.5 | 95.4±12.6# | 60.9±7.6 | 243.7±30.2# |
| | HDL-cholesterol | 65.0±2.8 | 77.7±2.4# | 75.0±3.7 | 106.3±3.7# |
| | Albumin | 4270±37 | 4259±57 | 4794±103 | 5161±79** |

| | | | | | |
|---|---|---|---|---|---|
| *: P < 0.05, in comparison to values of the control group | | | | | |
| **: P < 0.025, in comparison to values of the control group | | | | | |
| #: P < 0.001, in comparison to values of the control group | | | | | |

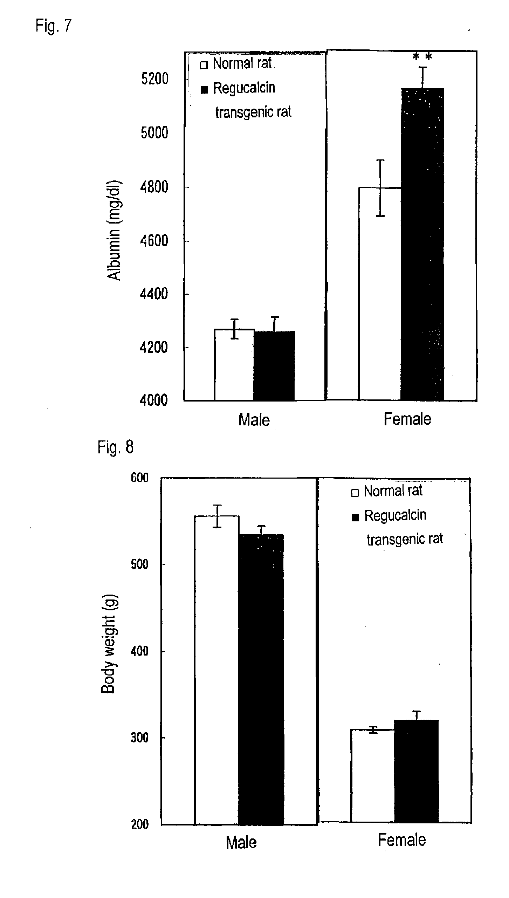

Based on the above knowledge, it has been revealed that regucalcin transgenic rats at the stage of senility develop hyperalbuminéa and hyperlipemia related to clinical condition of the liver. As shown in Fig. 8 and Table 1, the regucalcin transgenic rats did not exhibit significant changes in comparison to normal rats (wild-types) with regard to the body weight at 36 weeks of age.

### (Measurement results of calcium content in bone tissues)

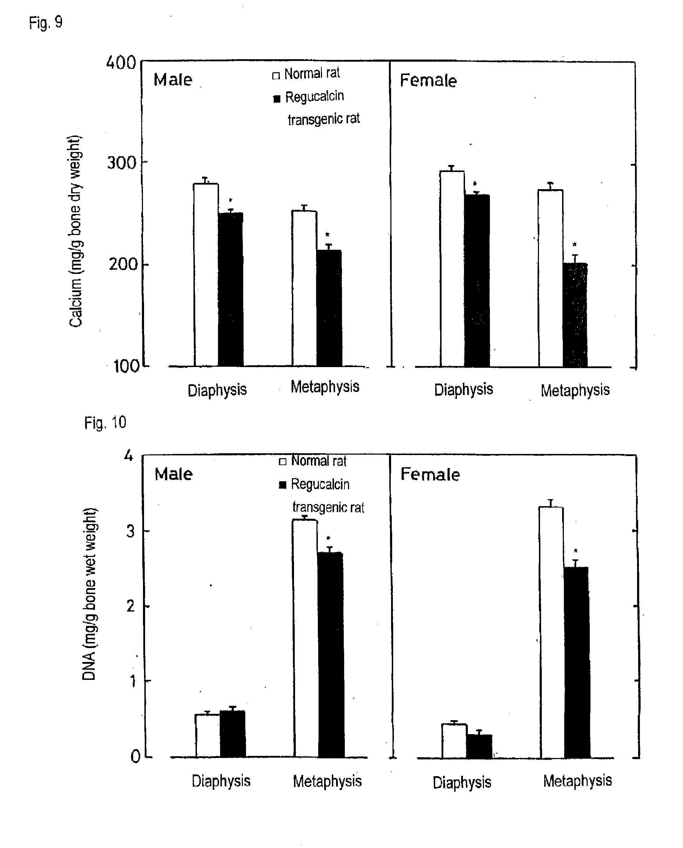

The measurement results of calcium content (mg/g bone dry weight) in femoral-diaphyseal and-metaphyseal tissues are shown in Fig. 9. From Fig. 9, it is observed that the calcium content in femoral diaphysis and metaphysis of 36-week-old regucalcin transgenic rats significantly decreases in both male and female, in comparison to those of normal rats (wild-types), revealing that the regucalcin transgenic rats develop bone loss also at the stage of senility. In Fig. 9, the symbol represents as follows: *: P < 0.01 (in comparison to values of the control group).

### (Measurement results of DNA content in bone tissues)

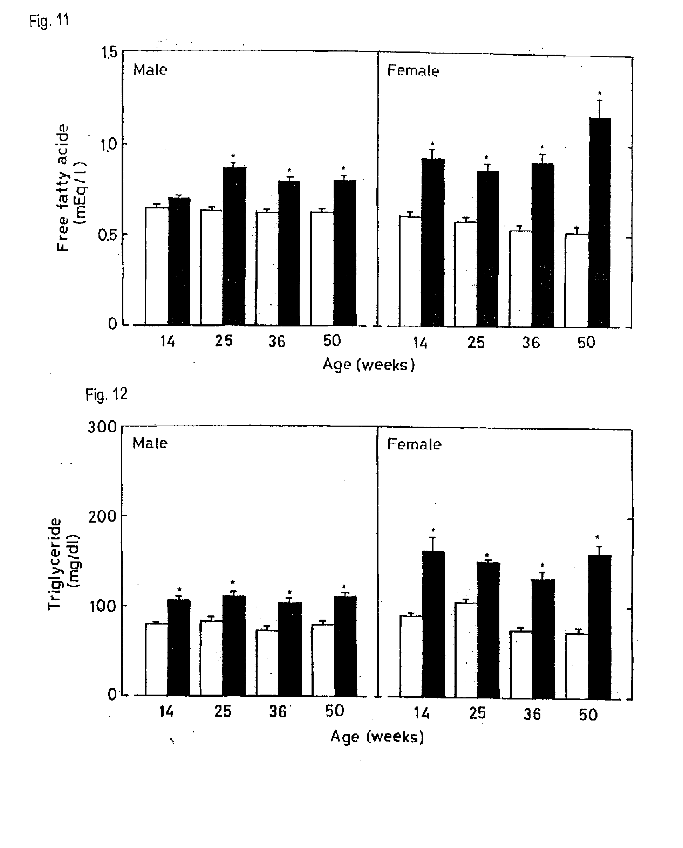

The measurement results of DNA content (an index of cell count) in femoral-diaphyseal and -metaphyseal tissues are shown in Fig. 2. From Fig. 10, it is observed that DNA content in the bone tissues significantly decreases particularly in metaphysis, and in male and female regucalcin transgenic rats, and it is considered that bone formation is suppressed. In Fig. 10, the symbol represents: *: P < 0.01 (in comparison to values of the control group).

### Example 3

### [Measurement of components in 14-, 25-, 36-, 50-week-old transgenic rats]

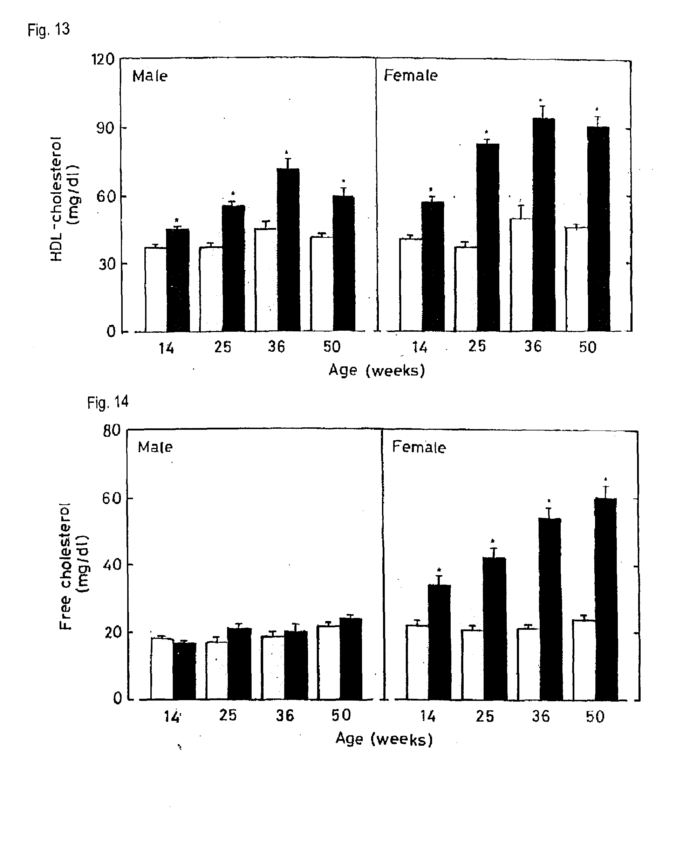

With the use of 14-, 25-, 36-, 50-week-old rats which overexpress regucalcin, the development of hyperlipemia/hyperalbuminea was examined. Each of the regucalcin transgenic rats (male and female homozygotes) and normal rats (male and female) was dissected at 14, 25, 36, or 50 weeks of age to collect the blood, and serum lipid concentration (free fatty acid, triglyceride, HDL-cholesterol, free cholesterol), and serum concentrations of calcium, inorganic phosphorus, zinc, glucose and urea nitrogen were measured in the same manner as in Example 2, then age-related changes in these components were examined respectively. The measurement kits "NEFA C-Test Wako" and "Urea Nitrogen B-Test Wako" made by Wako Pure Chemical Industries, Ltd. were used for the measurement of free fatty acid and urea nitrogen. The results are shown in Figs. 11 to 15 and Table 2. Each value in Figs. 11 to 15 and Table 2 shows mean value of 6 rats and standard error thereof. Further, in Figs. 11 to 15, the symbol represents as follows: *: P < 0.01 (in comparison to values of normal rats at respective weeks of age in the control group), and in Table 2, each symbol represents: ^{a}: P < 0.05, ^{b}: P < 0.01 (in comparison to values of normal rats at respective weeks of age in the control group).

Consequently, as shown in Figs. 11 to 14, it has been revealed that the elevated level of serum lipid concentrations (free fatty acid, triglyceride, HDL-cholesterol, free cholesterol) is observed in female rats that are 14 weeks of age or older, and that the elevation is significant in 50-week-old (1-year-old) rats.

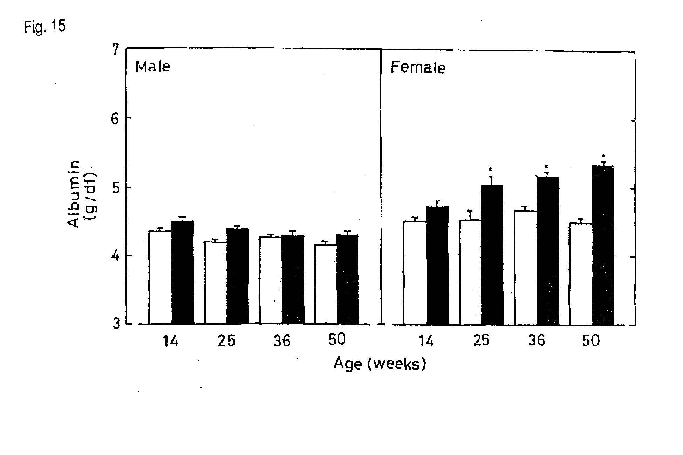

On the other hand, as shown in Fig. 15, it has been revealed that the elevated level of serum albumin concentration is observed in female rats that are 25 weeks of age or older.
It is assumed that the marked development of hyperlipemia and hyperalbuminea in female rats thus described is related to the fact that a regucalcin gene is localized on chromosome X.

**[Table 2]**

| Age-related changes in the serum components in regucalcin transgenic rats | | | | | | |
|---|---|---|---|---|---|---|
| Weeks of age | | Serum concentrations (mg/dl) | | | | |
| | | Calcium | Inorganic phosphorus | Zinc | Glucose | Urea nitrogen |
| 14 weeks | | | | | | |
| Male | Normal | 9.38±0.17 | 5.72±0.19 | 0.154±0.009 | 120.1±4.8 | 23.8±0.25 |
| | TG | 8.66±0.14 | 5.28±0.21 | 0.162±0.011 | 124.9±5.3 | 23.7±0.19 |
| Female | Normal | 8.19±0.04 | 4.86±0.19 | 0.147±0.004 | 125.0±5.4 | 23.5±0.96 |
| | TG | 8.80±0.09 | 5.60±0.18^{a} | 0.145±0.009 | 129.1±1.2 | 22.9±0.35 |
| 25 weeks | | | | | | |
| Male | Normal | 9.94±0.11 | 4.16±0.19 | 0.167±0.008 | 154.5±5.4 | 17.7±0.28 |
| | TG | 10.22±0.20 | 4.54±0.13 | 0.172±0.005 | 149.8±5.2 | 16.4±0.30 |
| Female | Normal | 9.89±0.16 | 3.23±0.09 | 0.166±0.005 | 138.6±3.7 | 19.5±0.81 |
| | TG | 10.10±0.18 | 3.96±0.21^{a} | 0.150±0.010 | 138.5±6.8 | 18.6±0.45 |
| 36 weeks | | | | | | |
| Male | Normal | 9.66±0.13 | 4.86±0.32 | 0.161±0.009 | 117.8±6.6 | 17.5±1.01 |
| | TG | 9.98±0.26 | 4.55±0.35 | 0.170±0.010 | 118.2±2.5 | 16.7±0.40 |
| Female | Normal | 10.46±0.56 | 3.33±0.15 | 0.195±0.018 | 129.4±4.4 | 20.1±0.53 |
| | TG | 10.76±0.58 | 4.00±0.32^{a} | 0.212±0.009 | 123.7±1.9 | 21.2±0.49 |
| 50 weeks | | | | | | |
| Male | Normal | 10.50±0.15 | 4.25±0.38 | 0.165±0.008 | 153.5±5.1 | 17.2±0.78 |
| | TG | 11.50±0.43^{b} | 4.25±0.20 | 0.165±0.008 | 156.7±6.1 | 20.1±1.15 |
| Female | Normal | 10.76±0.52 | 3.21±0.19 | 0.164±0.013 | 130.8±8.6 | 20.3±1.25 |
| | TG | 12.04±0.19^{b} | 2.98±0.18 | 0.177±0.010 | 147.9±3.2 | 22.3±1.26 |

| | | | | | | |
|---|---|---|---|---|---|---|
| Each of the regucalcin transgenic rats and normal rats was dissected at 14, 25, 36, or 50 weeks of age to collect blood. Each value is indicated as means ± standard error of 6 animals. | | | | | | |
| ^{a}:p<0.05, ^{b}:P < 0.01 (in comparison to those of normal rats) | | | | | | |

Further, as shown in Table 2, it has been found that the serum calcium concentration increases significantly in regucalcin transgenic rats (male, female) at 50 weeks of age. In addition, the serum inorganic phosphorus concentration increased significantly in female regucalcin transgenic rats at 14, 25, 36 weeks of age.

The serum concentrations of zinc, glucose and urea nitrogen of the regucalcin transgenic rats (male, female) at 14, 25, 36, and 50 weeks of age did not change significantly in comparison to those of normal rats.

As stated above, hyperlipemia is extremely characteristic in the regucalcin transgenic rats, and hyperalbuminea, particularly in female rats, is also a particular phenomenon.

Findings further supporting the usefulness of the regucalcin transgenic rats as an animal model for hyperlipemia/hyperalbuminea are obtained.

### Industrial Applicability

With the use of the hyperlipemia and/or hyperalbuminemia animal model of the present invention, it becomes possible to obtain fundamental knowledge of the onset mechanisms of hepatic diseases and hyperlipemia at the stage of advanced age, and moreover, the animal model can be advantageously used for the development of preventive/therapeutic drugs for diseases showing clinical examples of hyperlipemia and hyperalbuminea.

## Claims

1. A hyperlipemia and/or hyperalbuminemia animal model comprising a transgenic non-human animal into which a regucalcin gene is introduced and which overexpresses regucalcin.

2. The hyperlipemia and/or hyperalbuminemia animal model according to claim 1, which is obtained by raising the transgenic non-human animal to the stage of senility (advanced age) at which it exhibits a symptom of hyperlipemia and/or hyperalbuminemia.

3. The hyperlipemia and/or hyperalbuminemia animal model according to claim 1, which is obtained by raising the transgenic non-human animal (female) until it exhibits a symptom of hyperalbuminemia.

4. The hyperlipemia and/or hyperalbuminemia animal model according to any one of claims 1 to 3, wherein the non-human animal exhibits a bone disorder at the stage of senility (advanced age).

5. The hyperlipemia and/or hyperalbuminemia animal model according to any one of claims 1 to 4, which is a homozygote.

6. The hyperlipemia and/or hyperalbuminemia animal model according to any one of claims 1 to 5, wherein the non-human animal is a rat.

7. The hyperlipemia and/or hyperalbuminemia animal model according to claim 6, wherein the stage of senility (advanced age) means 36 to 50 weeks of age.

8. A method for using a transgenic non-human animal into which a regucalcin gene is introduced and which overexpresses regucalcin as an animal model for hyperlipemia and/or hyperalbuminemia.

9. The method according to claim 8, wherein the transgenic non-human animal is raised to the stage of senility (advanced age) and used as an animal model for hyperlipemia and/or hyperalbuminemia.

10. The method according to claim 8, wherein the transgenic non-human animal (female) is raised until it exhibits a symptom of hyperalbuminemia and used as an animal model for hyperlipemia and/or hyperalbuminemia.

11. The method according to any one of claims 8 to 10, wherein the non-human animal exhibits a bone disorder at the stage of senility (advanced age) .

12. The method according to any one of claims 8 to 11, which is a homozygote.

13. The method according to any one of claims 8 to 12, wherein the non-human animal is a rat.

14. The method according to claim 13, wherein the stage of senility (advanced age) means 36 to 50 weeks of age.

15. A method for screening a therapeutic drug for hyperlipemia and/or hyperalbuminemia comprising the steps of; administering a test substance to the hyperlipemia and/or hyperalbuminemia animal model according to any one of claims 1 to 7, and measuring/evaluating the amount of lipid and/or albumin in blood.

16. A method for screening a preventive drug for hyperlipemia and/or hyperalbuminemia comprising the steps of; administering a test substance to the hyperlipemia and/or hyperalbuminemia animal model according to any one of claims 1 to 7 before it reaches the stage of senility (advanced age) at which it exhibits a symptom of hyperlipemia and/or hyperalbuminemia, and measuring/evaluating the amount of lipid and/or albumin in blood after it reaches the stage of senility (advanced age).
